# EUROPÄISCHE PATENTANMELDUNG

(11) **EP 0 633 470 A2**
(43) Veröffentlichungstag der Anmeldung: **11.01.1995**
(21) Anmeldenummer: 94108268.7
(22) Anmeldetag: 28.05.1994
(51) Int. Cl.: G01N 33/15, G01N 33/00

(54) **Verfahren und Mittel zur Messung von Quecksilber (Hg), das aus dem Dentalwerkstoff Amalgam freigesetzt wird**

(30) Priorität: 10.07.1993 DE 4323072
(71) Anmelder: GSF-Forschungszentrum für Umwelt und Gesundheit, GmbH, D-85758 Oberschleissheim (DE)
(72) Erfinder: Halbach, Stefan, Dr., D-83209 Prien (DE)

(57) **Zusammenfassung**

Die Erfindung betrifft ein Verfahren und Mittel zur Messung von Quecksilber (Hg), das aus dem Dentalwerkstoff Amalgam freigesetzt wird.

Aufgabe der Erfindung ist es, dieses Verfahren so auszugestalten, daß bei einer Probenahme beide Hg-Formen erfaßt werden.

Gelöst wird diese Aufgabe durch Spülen der Mundhöhle eines Amalgamträgers mit einer ungiftigen Spülflüssigkeit, Aufnehmen des Spülflüssigkeit, nachdem dampfförmiges Hg (atomar) im unpolaren und ionisches Hg im polaren Lösungsmittel angereichert wurde, und Messen der jeweiligen Hg-Formen in den Einzellösungen.

## Beschreibung

Die Erfindung betrifft ein Verfahren und Mittel zur Messung von Quecksilber (Hg), das aus dem Dentalwerkstoff Amalgam freigesetzt wird.

Die in dem Dentalwerkstoff Amalgam enthaltenen Metalle Quecksilber, Silber, Kupfer und Zinn werden aus ihrer festen Bindung bekanntermaßen durch Abrieb beim Kauen und durch elektrochemische Korrosion in sehr geringen Mengen freigesetzt. Das giftigste unter diesen ist Quecksilber (Hg), weshalb in der Öffentlichkeit wiederholt ein Anwendungsverbot von Amalgam gefordert wurde. Dem werden vonseiten der Zahnmedizin die großen zahntechnischen Vorteile dieses Werkstoffs entgegengehalten. Die so entstehende Nutzen-Risiko-Abwägung hängt entscheidend davon ab, wieviel Hg aus den Füllungen in den Organismus gelangt. Das Quecksilber tritt nämlich sowohl in elementarer Form als Gas (Hg°) als auch in ionisierter Form (Hg²⁺) aus den Füllungen aus, daneben ist auch der Abrieb intakter Amalgampartikel möglich. Die beiden ersten Formen gelangen z. T. in den Blutkreislauf, so daß dieses Quecksilber zur Belastung des Organismus beiträgt, während die Partikel den Körper unverändert verlassen.

Aus Berglund, A., Pohl, L., Olsson, S., Bergman, M. (1988) Determination of the rate of release of intra-oral mercury vapor from amalgam. J. Dent. Res., 67, 1235-1242 ist bekannt, die Luft mit dem dampfförmigen Hg aus dem Mund in ein sog. Goldfilm-Meßgerät zu saugen, das die Hg-Menge anzeigt. Unabhängig von den Luftproben, jedoch nicht gleichzeitig mit diesen, können auch Speichelproben gewonnen und auf ionisiertes Hg untersucht werden. Dies ist aus Berglund, A. (1990) Estimation by a 24-hour study of the daily dose of intra-oral mercury vapor inhaled after release from dental amalgam. J. Dent. Res., 69, 1646-1651 bekannt.

Ein Nachteil bei der Absaugung der Luft aus dem Mundraum besteht darin, daß das Saugrohr in den halbgeöffneten Mund eingeführt wird und sich deshalb während des Absaugens die Hg°-haltige Luft mit nachströmender Hg-freier Außenluft vermischt. Dadurch erfolgt eine Verdünnung der Hg°-Konzentration in der untersuchten Luftprobe. Dieses Verfahren erfordert außerdem die Mitbestimmung des Volumens der Luftprobe (Durchflußmesser, Gassammler), auf das die in der Probe gefundene Hg-Menge bezogen werden muß. Bei der Messung der Quecksilberkonzentration im Speichel liegt ein Nachteil darin, daß das während eines bestimmten Zeitraums produzierte gesamte Speichelvolumen nicht bekannt ist, weshalb die in den Speichel insgesamt freigesetzte Hg-Menge nicht berechnet werden kann.

Alle Verfahren verfolgen das Ziel, die zeitabhängige Freisetzung von Quecksilber aus den Zahnfüllungen zu erfassen (Masse pro Zeit), woraus sich die täglich freigesetzte Menge errechnen läßt. Diese ist die entscheidende Kenngröße zur Abschätzung des vom Amalgam ausgehenden toxischen Gefährdungspotentials. Das Problem der bisherigen Verfahren stellt sich so dar, daS die Hg-Abgabe aus Amalgamfüllungen nur in nacheinander entnommenen Proben von unterschiedlicher Zustandsform gemessen wurde, was verschiedene Techniken der Probenahme und z. T. unterschiedliche Analysengeräte erforderlich macht. Dadurch wird die Vergleichbarkeit der Meßergebnisse eingeschränkt. Außerdem ist eine Berechnung der pro Zeiteinheit abgegebenen Hg²⁺-Menge anhand von Speichelproben nicht möglich.

Aufgabe der Erfindung ist es, Verfahren und Mittel zur Messung von Quecksilber, das aus dem Dentalwerkstoff Amalgan freigesetzt wird, zur Verfügung zu stellen, wobei bei einer Probenahme beide Hg-Formen erfaßt werden.

Gelöst wird diese Aufgabe durch die Merkmale der Patentansprüche 1, 3 und 6. Die Unteransprüche beschreiben vorteilhafte Ausgestaltungen der Erfindung.

Ein Vorteil des Verfahrens besteht darin, daß die Erfassung des gesamten in den Speichel freigesetzten Hg²⁺ dadurch ermöglicht wird, daß das unbekannte Speichelvolumen durch ein bekanntes an wäßriger NaCl-Lösung ersetzt wird. Die Cl-Anionen dienen dabei der Stabilisierung der Hg²⁺-Ionen. Wird der Mund eines Amalgamträgers mit einer Mischung mit dieser Lösungen ausgespült, können in einem Arbeitsgang beide Hg-Formen isoliert werden.

Das Verfahren macht sich die große Fettlöslichkeit von elementarem gasförmigem Quecksilber zunutze, um diese aus Amalgam freigesetzte Zustandsform von Hg zu messen. Allerdings kommen für die Anwendung am Patienten nur ungiftige und bei Zimmertemperatur flüssige Fette in Betracht. Außer den für Laborversuche zur Fettlöslichkeit von Hg° verwendeten Speiseölen wird auch dünnflüssiges Paraffin in pharmazeutischer Reinheit verwendet. Durch Beimengung einer wäßrigen Lösung zum Paraffin läßt sich gleichzeitig das aus Amalgam freigesetzte ionisierte Quecksilber auffangen, zu dessen Stabilisierung die wäßrige Lösung Cl⁻-Ionen in physiologischer Konzentration enthält. Die Konzentration von beiden Hg-Formen wird also in flüssigem Medium bestimmt, so daß die Analytik einheitlich in bekannter Weise im nassen Reduktionsverfahren in Verbindung mit der AAS durchgeführt wird. Auf zusätzliche Edelmetall-Kollektoren zur Adsorption von Hg° aus der Luft, bzw. auf das völlig andersartige Goldfilm-Meßgerät kann dabei verzichtet werden. Ein weiterer Vorteil ist die genaue Bemessung sowohl der Lösungsvolumina als auch der Zeitdauer der Mundspülung, womit anhand der in Paraffin und wäßriger Lösung gefundenen Konzentrationen die pro Zeiteinheit freigesetzte Menge der jeweiligen Hg-Form einfach und zuverlässig berechnet werden kann. Für den Patienten bringt das neue Verfahren eine Verbesserung dahingehend, daß er sich nur einmal der Prozedur zur Hg-Gewinnung aus dem Mundraum unterziehen muß. Da das Verfahren auch im Labor zur Untersuchung der Hg-Abgabe aus Amalgamprüfkörpern verwendbar ist, werden Übertragbarkeitsfehler beim Vergleich von in-vivo mit in-vitro Ergebnissen minimiert.

Die Erfindung wird im folgenden anhand eines Ausführungsbeispiels näher erläutert:
1. Der Patient spült den Mund zweimal mit Wasser gut aus, wodurch alles zuvor angesammelte Quecksilber aus der Mundhöhle entfernt wird.
2. Sofort anschließend spült er den Mund mit einer Spülflüssigkeit aus 15 ml Paraffin und 15 ml wäßriger 0,9 % NaCl-Lösung für zwei bis vier Minuten aus. Dabei soll die Zunge häufig, aber nicht zu heftig bewegt werden, um die Mischung gleichmäßig mit allen Amalgamflächen in Kontakt zu bringen.
3. Nach Ablauf der Spülzeit wird die gesamte Spülflüssigkeit in ein Zentrifugenröhrchen (50 ml Fassungsvermögen, Kunststoff, mit Schraubkappe) entleert und solange zentrifugiert, bis sich Wasser und Paraffin gut getrennt haben.
4. Analytische Bestimmung der Hg-Konzentration in einer Paraffin- und wäßrigen Probe mit üblichen Verfahren (Atomabsorptionsspektrometrie).
5. Berechnung der Hg-Freisetzungsrate (ng Hg/min):
   a) für Hg° (Paraffin):
      ng Hg°/min = (ng Hg/ml)_{Paraffin-Probe} x 15 ml/2 min;
   b) für Hg²⁺ (wäßrige Lösung):
      ng Hg²+/min = (ng Hg/ml)_{wäßr.Probe} x 15 ml/2 min.

Die Bestimmung der Hg-Freisetzungsrate nach 1. - 5. kann sowohl einige Stunden nach der letzten Mahlzeit erfolgen (sog. unstimuliertes Amalgam) als auch unmittelbar nach dem Kauen von Kaugummi (sog. stimuliertes Amalgam), wodurch die höhere Hg-Abgabe während der Mahlzeiten simuliert wird. Mit diesen Werten kann nach einem gängigen Schema auf die vom Organismus täglich aus den Amalgamfüllungen aufgenommene Hg-Menge hochgerechnet werden.

Für werkstoffkundliche Untersuchungen wird ein verschließbares Inkubationsgefäß mit einer Spülflüssigkeit nach 2. und einem Rührmagneten beschickt. Dieses wird dann in ein thermostatisiertes Wasserbad (37° C) gestellt, das auf einem laufenden Magnetrührer steht, und vorgewärmt. Anschließend wird die Amalgamprobe in das Inkubationsgefäß gegeben und dieses sofort verschlossen (Beginn der Inkubationszeit). Nach Ablauf der Inkubationszeit erfolgen die weiteren Schritte analog zu 3. - 5.

Anstelle des Paraffins können auch Speiseöle verwendet werden.

## Patentansprüche

1. Verfahren zur Messung von Quecksilber (Hg), das aus dem Dentalwerkstoff Amalgam freigesetzt wird, mit folgenden Verfahrensschritten:
a) Spülen der Mundhöhle eines Amalgamträgers mit einer ungiftigen Spülflüssigkeit, die aus einem unpolaren und einem polaren Lösungsmittel besteht, wobei die beiden Lösungsmittel nicht miteinander mischbar sind,
b) Aufnehmen der Spülflüssigkeit, nachdem dampfförmiges Hg (atomar) im unpolaren und ionisches Hg im polaren Lösungsmittel angereichert wurde,
c) Trennen der Spülflüssigkeit in seine beiden Bestandteile und
d) Messen der jeweiligen Hg-Formen in den Einzellösungen.

2. Verfahren nach Anspruch 1, dadurch gekennzeichnet, daß vor Schritt a) der Mund des Amalgamträgers mit Wasser ausgespült wird.

3. Verfahren zur Messung von Quecksilber (Hg), das aus dem Dentalwerkstoff Amalgam freigesetzt wird, mit folgenden Verfahrensschritten:
a) Inkubieren einer Amalgamprobe in einem Laborversuch in einer ungiftigen Spülflüssigkeit, die aus einem unpolaren und einem polaren Lösungsmittel besteht, wobei die beiden Lösungsmittel nicht miteinander mischbar sind,
b) Aufnehmen der Spülflüssigkeit, nachdem dampfförmiges Hg (atomar) im unpolaren und ionisches Hg im polaren Lösungsmittel angereichert wurde,
c) Trennen der Spülflüssigkeit in seine beiden Bestandteile und
d) Messen der jeweiligen Hg-Formen in den Einzellösungen.

4. Verfahren nach einem der Ansprüche 1 bis 3, dadurch gekennzeichnet, daS der Anteil des polaren Lösungsmittels in der Spülflüssigkeit zwischen 20 % und 80 % liegt.

5. Verfahren nach einem der Ansprüche 1 bis 3, dadurch gekennzeichnet, daß die Spülflüssigkeit zu gleichen Teilen aus polarem und unpolaren Lösungsmittel besteht.

6. Spülflüssigkeit bestehend aus
a) dünnflüssigem Paraffin in pharmazeutischer Reinheit oder Speiseöl als unpolare Komponente und
b) destilliertem Wasser mit 0,5 bis 2 % NaCl als polare Komponente.

7. Verwendung der Spülflüssigkeit nach Anspruch 6 für das Verfahren nach einem der Ansprüche 1 bis 5.
